# EUROPÄISCHE PATENTSCHRIFT

(11) **EP 1 937 630 B1**
(45) Veröffentlichungstag und Bekanntmachung des Hinweises auf die Patenterteilung: **03.08.2011**
(21) Anmeldenummer: 06806251.2
(22) Anmeldetag: 13.10.2006
(51) Int. Cl.: C07C 309/24, C07C 407/00, C07C 409/16

(54) **VERFAHREN ZUR HERSTELLUNG ORGANISCHER PEROXIDE MITTELS MIKROREAKTIONSTECHNIK**
METHOD FOR THE PRODUCTION OF ORGANIC PEROXIDES BY MEANS OF A MICROREACTION TECHNIQUE
PROCEDE DE PRODUCTION DE PEROXYDES ORGANIQUES PAR LA TECHNIQUE DE MICROREACTION

(30) Priorität: 14.10.2005 DE 102005049294
(43) Veröffentlichungstag der Anmeldung: 02.07.2008
(73) Patentinhaber: Ehrfeld Mikrotechnik BTS GmbH, 55234 Wendelsheim (DE); Pergan Hilfsstoffe Für Industrielle Prozesse Gmbh, 46395 Bocholt (DE)
(72) Erfinder: AZZAWI, Alexander, 55129 Mainz (DE); MEHESCH, Hans-Ernst, 45134 Essen (DE); VON ZADOW, Edgar, 46342 Velen (DE); KIRSCH, Stefan, 06464 Quedlinburg (DE); WORMLAND, Bernhard, 46395 Bocholt (DE); SONDERMANN, Martin, 46414 Rhede (DE)
(74) Vertreter: Lütjens, Henning
(86) Internationale Anmeldenummer: PCT/EP2006/009898
(87) Internationale Veröffentlichungsnummer: WO 2007/042313

(56) Entgegenhaltungen:
- WO-A-2005/079964
- US-A1- 2003 055 293
- US-B1- 6 268 523

## Beschreibung

Gegenstand der Erfindung ist ein Verfahren zur effizienten und sicheren Herstellung von organischen Peroxiden, vorzugsweise Dialkylperoxiden, Peroxycarbonsäuren, Peroxycarbonsäureestern, Diacylperoxiden, Peroxycarbonatestem, Peroxydicarbonaten, Ketonperoxiden und Perketalen, mit Hilfe mindestens eines statischen Mikromischers und eine Vorrichtung zur Durchführung des Verfahrens.

Organische Peroxide sind sehr reaktive chemische Substanzen. Da sie leicht in äußerst aktive Radikale und Sauerstoff zerfallen, werden sie als Initiatoren in der Kunststoff bzw. Kautschukindustrie eingesetzt. Anwendungsgebiete der organischen Peroxide sind die Polymerisation von Monomeren zur Kunststoffherstellung, die Vernetzung und die Modifikation von Polymeren sowie die Härtung von Polyesterharzen. Ferner werden organische Peroxide als Oxidationsmittel in medizinischen Präparaten und für komplizierte chemische Synthesen verwendet.

Ein wesentliches Merkmal organischer Peroxide ist die SADT (Self Accelerating Decomposition Temperature). Sie ist die tiefste Temperatur, bei der eine selbst beschleunigende Zersetzung in der Transportverpackung auftreten kann. Eine gefährliche selbstbeschleunigende Zersetzungsreaktion, unter ungünstigen Umständen Explosion oder Feuer, kann durch thermische Zersetzung bei oder oberhalb der angegebenen Temperatur hervorgerufen werden. Ein Kontakt mit nicht verträglichen Substanzen sowie eine erhöhte mechanische Beanspruchung kann Zersetzung bei oder unterhalb der SADT hervorrufen.

Organische Peroxide werden heutzutage durch kontinuierliche oder diskontinuierliche Verfahren hergestellt (Chem. Ztg. 98 (tg. 12), 583 (1974), W. Mayr. Ullmann's encyclopedia of industrial chemistry, 6.Edition, Vol.25, 463 (2002)), DE 698 12 430 T2, DE 69904337 T2 oder auch US-A 6 268 523. Ein typisches Beispiel ist die Herstellung von tert.-Butylperoxy-2-ethlyhexanoat. Dieses organische Peroxid wird aus tert.-Butylhydroperoxid und 2-Ethyl-hexansäurechlorid bei einer Temperatur unterhalb von 35°C (= SADT) und einer durchschnittlichen Verweilzeit von bis zu 2 Stunden bei einem 500 kg Reaktionsansatz in einem Rührkessel hergestellt. Im Einzelnen sind nachstehende beispielhafte Reaktionsschemata für die Herstellung einzelner Peroxidklassen relevant:

Aus Säurechloriden und Wasserstoffperoxid entstehen Diacylperoxide:

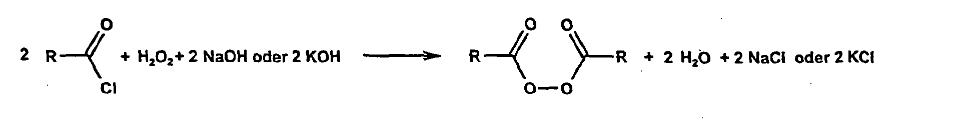

Aus Chlorformiaten und Wasserstoffperoxid entstehen Peroxydicarbonate:

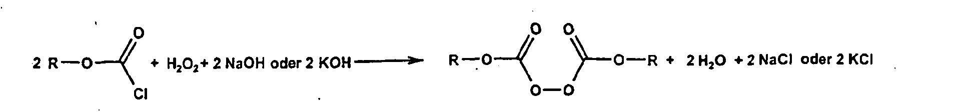

Aus Säurechloriden und organischen Hydroperoxiden entstehen Peroxyester:

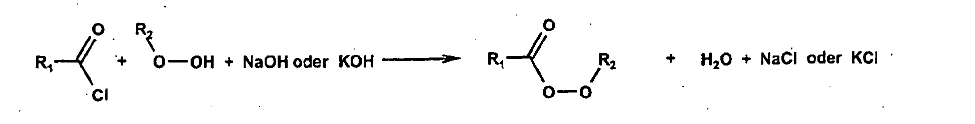

Aus Chlorformiaten und organischen Hydroperoxiden entstehen Percarbonatester: 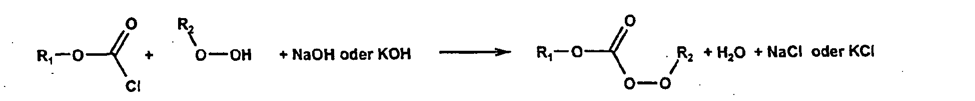 wobei R für einen beliebigen organischen Rest steht.

Ein wesentlicher Parameter bei der Herstellung organischer Peroxide ist die optimale Temperaturführung unter Einhaltung der erforderlichen Verweilzeit für die Reaktion. Erfahrungsgemäß liegt die erforderliche Reaktionstemperatur im Bereich der vorgenannten SADT, so dass ein lokales Überschreiten der Reaktionstemperatur im Reaktor, das zu einer unkontrollierbaren Zersetzung der Reaktionsmischung bzw. der Reaktionsprodukte führen kann, verhindert werden muss. Dies führt ebenso zu langen Reaktionszeiten. Bei den bekannten Herstellungsverfahren müssen demnach große Mengen, einige hundert Liter, des sehr reaktiven und explosionsgefährlichen Reaktionsgemisches unter maximaler Turbulenz in Behältern oder sonstigen Reaktionssystemen vorgehalten werden. Diese Vorgehensweise erfordert einen nicht unerheblichen Aufwand an speziellen Sicherheitseinrichtungen, wie z.B. im Bereich der Temperatur- und Turbulenzüberwachung.

Außerdem muss die Reaktion zur Erhöhung der Reaktionssicherheit in großer Verdünnung durchgeführt werden. Dadurch entsteht ein deutlicher Mehraufwand an Verdünnungsmitteleinsatz und entsprechend erforderlichen nachgeschalteten Abtrennungs-, Reinigungs- und Aufbereitungsverfahren. Zudem wird dadurch die Reaktion als auch der gesamte Herstellungsprozess verlangsamt. Weiterhin handelt sich bei nahezu allen Herstellungsmethoden um eine Zweiphasenreaktion, da die Reaktanden nicht vollständig miteinander mischbar sind. Um eine ausreichende Reaktionsgeschwindigkeit zu erzielen ist eine intensive feindisperse Durchmischung der beiden Phasen nötig. Dieses kann z.B. in einem herkömmlichen Rührkesselreaktor nur unzureichend gewährleistet werden. Die Herstellung in sonstigen statischen Mischern bzw. Rohrreaktoren ist aus Gründen der Verdämmung der explosionsfähigen organischen Peroxide auch unter Einbau von Druckentlastungseinrichtungen nicht empfehlenswert.

Aus DE 69618646 T2 sind kontinuierliche und diskontinuierliche Verfahren zur Herstellung von Acylperoxiden bekannt. Hierbei soll ein heftiges Aufrühren der Edukte mittels Strahl-, Statik- oder auch Ultraschallmischer Probleme der Stabilität der Reaktionsmischungen umgehen.

Bei den kontinuierlichen Verfahren können dabei Ausbeuten bis maximal 95,5 % erreicht werden, während bei den diskontinuierlichen Verfahren der Vorteil bei kürzeren Reaktionszeiten liegt, was aber zu Lasten der Produktausbeute geht.

Demnach ist im Stand der Technik kein Verfahren bekannt, dass die Herstellung organischer Peroxide ohne die zuvor beschriebenen Nachteile ermöglicht, d.h. eine sichere und schnelle Herstellung in hoher Ausbeute.

Es stellte sich deshalb die Aufgabe ein Verfahren zu finden, bei dem eine sichere Prozessführung bei einer geringen Verdünnung und maximal feindisperser Durchmischung der Reaktanden möglich ist. Zudem sollte auch eine für dieses Verfahren geeignete Vorrichtung zur Verfügung gestellt werden. Die Reaktanden müssen hierbei unter exakter Temperaturführung und -kontrolle miteinander zu Reaktion gebracht werden. Dabei ist zu beachten, dass nicht alle Reaktanden vollständig miteinander mischbar sind und daher für eine schnelle Reaktion die beiden Phasen ständig miteinander intensiv vermischt werden müssen. Des weiteren gilt es, die nachgeschalteten Phasentrennungs-, Reinigungs- und Aufbereitungsverfahren unter Optimierung der vorgenannten Parameter im Bereich der Prozess- und Sicherheitstechnik durchzuführen.

Überraschenderweise wurde nun gefunden, dass sich die Nachteile der Verfahren des Standes der Technik dadurch beseitigen lassen, dass die Herstellung von organischen Peroxiden, vorzugsweise Dialkylperoxiden, Peroxycarbonsäuren, Peroxycarbonsäureestern, Diacylperoxiden, Peroxycarbonatestem, Peroxydicarbonaten, Ketonperoxiden und Perketalen, in mindestens einem statischen Mikromischer durchgeführt wird.

Durch den Einsatz statischer Mikromischer konnte die Sicherheit des Herstellungsprozesses deutlich erhöht werden. In der eingesetzten Vorrichtung sind dabei kleine Mengen pro Volumeneinheit der problematischen Reaktions- bzw. Aufarbeitungsmischung zu handhaben und die genaue Temperaturkontrolle unterbindet ein unerwünschtes Ansteigen der Temperatur. Zudem wird die Effizienz des Prozesses gesteigert, da aufgrund der sehr hohen Wärmeaustauschfläche pro Volumeneinheit mit höher konzentrierten Reaktions- und Aufbereitungsmischungen gearbeitet und die Temperatur erhöht werden kann, ohne dass die zu erwartende größere Reaktionswärme zu sicherheitstechnischen Problemen führt. Überraschenderweise führen die deutlich konzentrierteren Verhältnisse in der Mikroreaktionsanlage nicht zu Problemen durch intermediär gebildete feste Zwischenprodukte aus den flüssigen Edukten, wie dies bei herkömmlichen Verfahren der Fall ist, weil diese Zwischenprodukte sehr schnell weiter zu den flüssigen Produkten umgesetzt werden. Sofern als Produkte der Reaktion Feststoffe gebildet werden, sind spezielle Mikroreaktoren zu verwenden, die gegen Verstopfung unempfindlich sind. Die intensive Vermischung der Phasen führt zudem zu einer Beschleunigung der Herstellungs- und Aufarbeitungsreaktionen sowie zu einer verbesserten Reaktionsumsetzung und Ausbeutesteigerung der Hauptreaktion. Die Reaktions- und Verfahrensführung in der Mikroreaktionsanlage ist herkömmlichen Methoden zur Herstellung der Produkte in Effizienz und Sicherheitstechnik weit überlegen.

Das erfindungsgemäße Verfahren ermöglicht zudem durch die Kombination der eigentlichen Reaktion mit der Aufbereitung und der Trocknung des schwer zu handhabenden Produktes auf einfache Weise eine sichere und effektive Umsetzung.

Gegenstand der vorliegenden Erfindung ist daher ein Verfahren zur Herstellung von organischen Peroxiden unter Einsatz von Wasserstoffperoxid oder Hydroperoxid, mindestens einer Base oder Säure und mindestens eines Ketons, Alkohols, Säurechlorids /-anhydrids und/oder Chlorformiats, wobei dieses in mindestens einem statischen Mikromischer durchgeführt wird.

Bei den organischen Peroxiden handelt es sich vorzugsweise um Dialkylperoxide (R₁-O-O-R₂), wie z.B. Di-tert.-butylperoxid, Di-(2-tert.-butylperoxyisopropyl)-benzen oder Dicumylperoxid, Peroxycarbonsäuren (R₁-C(O)-O-OH), wie z.B. Peroxyessigsäure, Peroxycarbonsäureester (R₁-C(O)-O-O-R₂), wie z.B. tert.-Butylperoxypivalat, tert.-Butylperoxy-2-ethylhexanoat, Diacylperoxide (R₁-C(O)-O-O-C(O)-R₂), wie z.B. Dibenzoylperoxid, Di-(3,5,5-trimethylhexanoyl)-peroxid, Peroxycarbonatester (R₁O-C(O)-O-O-R₂), wie z.B. tert.-Butylperoxyisopropylcarbonat, tert.-Butyl-peroxy-2-ethylhexylcarbonat, Peroxydicarbonate (R₁O-C(O)-O-O-C(O)-R₂,), wie z.B. Di-(4-tert.-butylcyclohexyl)-peroxydicarbonat, Di-(2-ethylhexyl)-peroxydicarbonat oder Dicetylperoxydicarbonat, Ketonperoxide, wie z.B. Cyclohexanonperoxid, Methylisobutylketonperoxid oder Methylethylketonperoxid und/oder Perketale, wie z.B. 2,2-Bis-(tert.-butylperoxy)-butan, 1,1-Ditert.-butylperoxy-3,3,5-trimethylcyclohexan oder 1,1-Bis-(tert.-butylperoxy)-cyclohexan, wobei R₁ und R₂ in allen Fällen beliebige organische Reste darstellen.

In einer bevorzugten Ausführungsform der Erfindung werden zwei oder mehr Reaktanden in einem oder mehreren mikrostrukturierten statischen Mischern intensiv durchmischt, die Reaktionsmischung in einem oder mehreren mikrostrukturierten Wärmetauschern ggf. an verschiedenen Prozesspunkten temperiert, durchströmt dann ein oder mehrere Verweilvolumina zur vollständigen Durchführung der Reaktion und das Verfahren wird vorzugsweise kombiniert mit der Aufarbeitung des Produktes und der abschließenden Trocknung des organischen Peroxides.

Als Hydroperoxide im Sinne der Erfindung sind alle gängigen bekannten Verbindungen einsetzbar, so z.B. Alkylhydroperoxide, wie tert.-Butylhydroperoxid oder Cumolhydroperoxid. Diese Edukte sind käuflich erhältlich oder lassen sich nach den bekannten Oxidationsverfahren, wie z.B. der Oxidation von Cumol mit Sauerstoff zur Herstellung von Cumolhydroperoxid oder der säurekatalysierten Oxidation des entsprechenden Alkohols mit Wasserstoffperoxid herstellen.

Als Base eignen sich ebenfalls alle im Stand der Technik bekannten Basen. Bevorzugt sind NaOH, KOH und/oder Ca(OH)₂ oder Imidazole, wie z.B. Methylimidazol.

Säuren im Sinne der Erfindung sind alle bekannten organischen und anorganischen Säuren. Bevorzugt dabei sind Schwefelsäure, Essigsäure oder Salzsäure.

Als Ketone können im erfindungsgemäßen Verfahren ebenfalls alle dem Fachmann bekannten Ketone eingesetzt werden. Bevorzugt dabei sind 3,3,5-Trimethylcyclohexanon, Methylethylketon und Methylisobutylketon.

Als Alkohole im Sinne der Erfindung sind auch alle gängigen Verbindungen einsetzbar. Bevorzugt dabei sind Methanol, Ethanol, tert.- Butanol, 2-Phenylpropan-2-ol aber auch Diole, wie z.B. Bis (α-hydroxyisopropyl)-benzol.

Die Art der für das erfindungsgemäße Verfahren eingesetzten Säurechloride ist ebenfalls nicht limitiert. Bevorzugt sind beispielsweise 2-Ethylhexansäurechlorid, 3,5,5-Trimethylhexanoylchlorid oder Benzoylchlorid.

Ebenfalls einsetzbar sind alle bekannten Säureanhydride, wie z.B. Essigsäureanhydrid oder Maleinsäureanhydrid.

Als Chlorformiate im Sinne der Erfindung können ebenfalls alle im Stand der Technik bekannten Verbindungen eingesetzt werden. Bevorzugt dabei sind beispielsweise 2-Ethylhexylchlorformiat, Isopropylchlorformiat oder n-Butylchlorformiat.

Die Konzentrationen der eingesetzten Agenzien können dabei stark variieren.

Bevorzugt sind für die Basen 10 bis 50 für die organischen Peroxidkomponenten 70 bis 100 %, für H₂O₂ 30 bis 70 %.

In einer bevorzugten Ausführungsform der Erfindung können Phlegmatisierungsmittel bzw. Lösemittel beigefügt werden. Hierfür eignen sich besonders Isododecan, Weißöl oder Phthalate wie Diisobutylphthalat. Weitere Additive und Hilfsmittel, wie z.B. Emulgatoren, können ebenfalls den Edukten beigefügt werden.

Für das erfindungsgemäße Verfahren können alle bekannten statischen Mikromischer eingesetzt werden. In einer bevorzugten Ausführungsform der Erfindung werden dabei als statische Mischer Multilaminationsmischer, Split- und Recombinemischer oder auch Mischer mit einer Querschnittsverengung eingesetzt. Hierbei werden vorzugsweise statische Mischer eingesetzt, die kontinuierlich durchströmt werden. Darunter fallen beispielsweise Stapelmischer (gemäß DE 202 06 371 U1) oder Schlitzplattenmischer (gemäß WO 2004 052518 A2) oder auch Kammmischer (gemäß DE 202 09 009 U1), wie sie z.B. bei Ehrfeld Mikrotechnik BTS GmbH angeboten werden. Hierbei handelt es sich um Multilaminationsmischer, bei denen die beiden zu vermischenden Fluidströme in eine Vielzahl von dünnen Lamellen oder Filmen aufgefächert werden und diese Lamellen anschließend abwechselnd ineinander gekämmt werden, so dass es durch Diffusion und Sekundärströmungen zu einer schnellen Vermischung kommt.

Ebenfalls möglich sind V-Typ-Mischer, wie sie u.a. beim Forschungszentrum Karlsruhe angeboten werden, Split- und Recombinemischer, wie z.B. Kaskadenmischer oder Rautenmischer wie sie bei Ehrfeld Mikrotechnik BTS GmbH angeboten werden oder Caterpillarmischer, z.B. erhältlich am Institut für Mikrotechnik Mainz, in dem die zu vermischenden Produktströme in kleinere Ströme aufgeteilt und diese kleinen Ströme wiederholt zusammengeführt und aufgeteilt werden, oder auch Mischer mit einer Querschnittsverengung, wie Fokusmischer oder Zyklonmischer, oder auch Jetmischer (gemäß EP 1 165 224 B1), z.B. erhältlich bei der Firma Synthesechemie und Prallstrahlmischer oder Ventilmischer. (wie in WO 2005/079964 A1 beschrieben) erhältlich bei Ehrfeld Mikrotechnik BTS GmbH.

In einer bevorzugten Ausführungsform der Erfindung werden Mikromischer mit Kanalweiten von kleiner als 500 µm, bevorzugt kleiner 200 µm, besonders bevorzugt kleiner 100 µm eingesetzt.

In derartige Mikromischer werden die Reaktandmischungen vorzugsweise mittels präziser und pulsationsarmer Pumpen, wie z.B. HPLC-Pumpen oder Spritzenpumpen, zudosiert. Besonders bevorzugt wird anschließend die so vermischte Reaktionsmischung durch eine Anordnung von mikrostrukturierten Wärmetauschern und Verweilvolumen mit vorgegebener Verweilzeit geleitet. Bevorzugt ist die Anordnung so gestaltet, dass der Temperaturverlauf im strömenden Reaktionsgemisch entlang der Strömungsrichtung durch die Abfolge von Wärmetauschern und Verweilerstrecken einstellbar ist.

Für das erfindungsgemäße Verfahren werden vorzugsweise alle bekannten mikrostrukturierten Wärmetauscher eingesetzt. Bekannt sind Mikro-Wärmetauscher, die aus gestapelten, mit vielen Mikrokanälen versehenen und miteinander verschweißten dünnen Platten bestehen. Die Grundgeometrie solcher Wärmetauscher ist vorzugsweise annähernd würfelförmig, wobei bei einer Kantenlänge von beispielsweise 3 cm Wärmeübertragungsleistungen im Bereich von 100 kW erzielt werden können, wie dies beispielhaft beschrieben ist in 2nd International Conference on Microreaction Technology, Tropical Conference Preprints, (ISBN 0-8169-9945-7, S. 88-95).

Solche mikrostrukturierten Wärmetauscher sind z.B. Kreuzstrom- oder Gegenstrom-Plattenwärmetauscher, wie in DE 37 09 278 A1 beschrieben, und wie sie z.B. bei der Ehrfeld Mikrotechnik BTS GmbH erhältlich sind. Aufgrund ihrer langen und sehr engen Kanäle sind jedoch diese Wärmetauscher in hohem Maß empfindlich gegenüber festen Partikeln, da bereits kleine Partikel im Reaktionsmedium diese Kanäle verstopfen können. Somit kommen diese Wärmetauscher bevorzugt zum Einsatz, wenn an der vorgesehenen Stelle im Prozess das Auftreten von Partikeln ausgeschlossen werden kann.

Muss mit dem Auftreten von Partikeln gerechnet werden, so kommen bevorzugt mikrostrukturierte Wärmetauscher zum Einsatz, deren engste Durchlassstelle so breit ist, dass die Partikel ungehindert passieren können. Ein solcher Wärmetauscher ist beispielsweise das Rohr-Temperiermodul der Ehrfeld Mikrotechnik BTS GmbH. Das Rohr-Temperiermodul besteht aus konzentrischen Rohren, die vom Fluid in einer Gegenstrom-Konfiguration durchströmt werden. Diese Anordnung wird vom Temperiermedium sowohl im Kern, als auch im Außenbereich durchströmt. Dieser Aufbau entspricht prinzipiell der Funktionsweise eines Intensivkühlers.

In einer bevorzugten Ausführungsform des erfindungsgemäßen Verfahrens wird der statische Mikromischer kontinuierlich durchströmt, die Reaktionsmischung mittels Wärmetauscher auf die geeignete Temperatur gebracht und anschließend gegebenenfalls die Reaktionsmischung in ein temperierbares Verweilvolumen eingespeist, dort eine durch das Verweilvolumen und die Fließgeschwindigkeit der Reaktionsmischung vorbestimmte Zeit verbleibt, wobei in diesem Verweilvolumen die Ausgangsstoffe, die gegebenenfalls als nicht mischbare Phasen vorliegen, ständig miteinander intensiv vermischt werden.

Als Wärmetauscher für das erfindungsgemäße Verfahren kommen Vorrichtungen in Frage, bei denen in das vom Reaktionsgemisch durchströmte Gehäuse senkrecht zur Strömung ausgerichtete Röhrchen eingebaut sind, die von einem Wärmeträgermedium durchströmt oder elektrisch beheizt werden. Zur Erhöhung des Wärmeübergangs werden solche Röhrchen in Richtung des Hauptstroms zweckmäßigerweise gegeneinander versetzt in mehreren Ebenen angeordnet. Bei Wärmetauscher-Modulen, die für wässrige Reaktionsmedien und Volumenströme im Bereich von 1000 L/h ausgelegt sind und eine Gehäusequerschnittsfläche von etwa 100 cm² besitzen, werden vorteilhaft Röhrchen mit einem Außendürchmesser im Bereich von einem Millimeter eingesetzt, die in einem Abstand von weniger als einem Millimeter angeordnet sind. Solche Hochleistungs-Mikrowärmetauscher erreichen Übertragungsleistungen von einigen 10 kW, weisen geringe Druckverluste auf und sind überdies leicht zu reinigen.

Bei einem anderen Typ eines Hochleistungs-Mikrowärmetauschers werden in das vom Reaktionsgemisch durchströmte Gehäuse plattenförmige Körper eingebaut, die elektrisch beheizt oder als Hohlkörper von einem Wärmeträgermedium durchströmt werden. Solche Mikroplattenwärmetauscher haben typischerweise Plattenabstände im Submillimeterbereich, wobei die Platten einfach demontiert und gereinigt werden können. Wie bei dem oben beschriebenen Mikrorohrbündelwärmetauscher werden auch hier bei entsprechenden Randbedingungen Wärmeübertragungsleistungen von einigen 10 kW erreicht.

Bei dem Verweilvolumen oder auch der Verweilstruktur im Sinne der Erfindung handelt es sich um abgegrenzte Volumina, die aufgrund ihres inneren Volumens in einer vorgegebenen Zeit durchströmt werden können, wie z.B. Kapillaren oder auch mikrostrukturierte statische Mischer. Es können unterschiedliche Verweilvolumina eingesetzt werden, die sich jeweils durch eine möglichst enge Verweilzeitverteilung auszeichnen und geringe Totvolumina aufweisen. Vorzugsweise können diese Verweilvolumina temperiert werden, indem elektrische Heizungen oder Kühlvorrichtungen angebracht sind oder indem ein Temperierfluid das Verweilvolumen umströmt.

Im einfachsten Fall besteht das Verweilvolumen aus einer Kapillare vorgegebener Länge, aber auch andere gleichmäßig durchströmte Volumina oder Anordnungen können eingesetzt werden.

Besonders geeignet ist eine Anordnung aus Verweilvolumina, bei der zwischen einfach durchströmten Volumina jeweils ein statischer, vorzugsweise ein mikrostrukturierter statischer Mischer zur Durchmischung von mehrphasigen Gemischen angebracht ist.

In einer weiteren bevorzugten Ausführungsform der Erfindung erfolgt in dieser Verweilstruktur eine ständige feindisperse Durchmischung der nicht mischbaren Reaktanden mittels eines oder mehrerer statischer Mischer oder durch hochfrequente mechanische Einwirkung, z.B. von Ultraschall auf eine definierte Verweilstruktur oder durch eine Kombination aus einem oder mehreren statischen Mischern und einer hochfrequenten mechanischen Einwirkung. Der Begriff hochfrequent umfasst dabei Frequenzen im Bereich von 10 kHz bis 20 MHz.

An geeigneten Stellen im Reaktionsbereich werden vorzugsweise Temperatursensoren und mikrostrukturierte Wärmetauscher zur genauen Kontrolle und Einhaltung der Reaktions- und Verarbeitungstemperatur eingesetzt. Nur durch den Einsatz von mikrostrukturierten Wärmetauschern kann sichergestellt werden, dass die Reaktionsmischung die kritische Zersetzungstemperatur nicht überschreitet, auch wenn die Reaktionstemperatur nahe dieser Zersetzungstemperatur liegt. Ebenso besonders geeignet ist eine Anordnung, bei der die Verweilvolumina durch direkt angebrachte Ultraschallschwinger oder durch Eintauchen der Verweilvolumina in ein Bad mit hochfrequenten Schwingungen oder durch Aufsetzen von Piezomodulen durchsetzt werden können.

Ebenfalls können Verweilstrukturen eingesetzt werden, bei denen das Gemisch im Kreislauf analog zu einem Schlaufenreaktor umgepumpt wird, wobei ggf. ein oder mehrere mikrostrukturierte statische Mikromischer in den Kreislauf eingefügt sind.

Der Temperaturverlauf im strömenden Reaktionsgemisch entlang der Strömungsrichtung wird dabei vorzugsweise durch eine Abfolge von Wärmetauschern und Verweilvolumina eingestellt.

Die Reaktionstemperatur hängt dabei von den eingesetzten Edukten ab und liegt vorzugsweise im Bereich von 10 - 70 °C.

Nach dem Durchlaufen der Verweilvolumina ist es vorteilhaft, wenn das Peroxid einer Aufarbeitung zugeführt wird. Diese ist vorzugsweise analog zu der Reaktionsapparatetechnik aufgebaut.

Die Aufbereitung des herzustellenden organischen Peroxides unterteilt sich dabei vorzugsweise in den Bereich der Abtrennung des organischen Peroxides von der wässrigen Mutterlauge und der Reinigung des organischen Peroxides und der anschließenden Phasentrennung. Bei der Reinigung werden das Rohprodukt und die Waschlösungen in definierten Mengenströmen einem Mikromischer, vorzugsweise einem statischen Mikromischer, zugeführt und dort intensiv vermischt. Anschließend wird diese Mischung vorzugsweise in ein Verweilvolumen, das vorzugsweise temperierbar ist, eingespeist, wo sie eine durch das Volumen der Verweilstruktur und die Fließgeschwindigkeit der Reaktionsmischung vorbestimmte Zeit verbleibt. Zusätzlich wird in dieser Verweilstruktur eine ständige feindisperse Durchmischung der nicht mischbaren Reaktanden mittels eines oder mehrerer statischer Mischer oder durch hochfrequente Einwirkung, wie z.B. von Ultraschall auf eine definierte Verweilstruktur oder durch eine Kombination aus einem oder mehreren statischen Mischern und einer hochfrequenten Einwirkung sichergestellt.

Nach der durchgeführten Wäsche wird die gebildete Zwangsemulsion vorzugsweise in einem Separator, vornehmlich Mikroseparationsmodul in die jeweiligen Phasen zur weiteren Aufbereitung aufgetrennt.

Nach vorgenannter Aufbereitung wird vorzugsweise das Wasser aus dem flüssigen organischen Peroxid in einem Trocknungsprozess entfernt. Diese Trocknung kann gemäß dem Stand der Technik mittels Trocknungsmittel, wie z.B. Zeolithen, Magnesiumsulfat, Magnesiumchlorid o.a. oder mittels entfeuchteter Luft oder einem sonstigen trockenem Gas im Gegen- oder Kreuzstrom vorgenommen werden. Bei der hier vorzunehmenden Trocknung wird das organische Peroxid in definierten Mengenströmen einem Mikroextraktionsmischer, vorzugsweise einem statischen Mikroextraktionsmischer, im Gegenstrom zu entfeuchteter Luft zugeführt und dort intensiv vermischt. Anschließend wird die wasserhaltige Luft bzw. das wasserhaltige Gas weiteren Aufarbeitungsschritten zugeführt und das hergestellte organische Peroxide der Abfüllung und Konfektionierung zugeleitet.

Die Erfindung wird durch die nachstehenden Abbildungen und die folgenden Beispiele näher erläutert, ohne dass diese darauf beschränkt ist.

### Ausführungsbeispiele

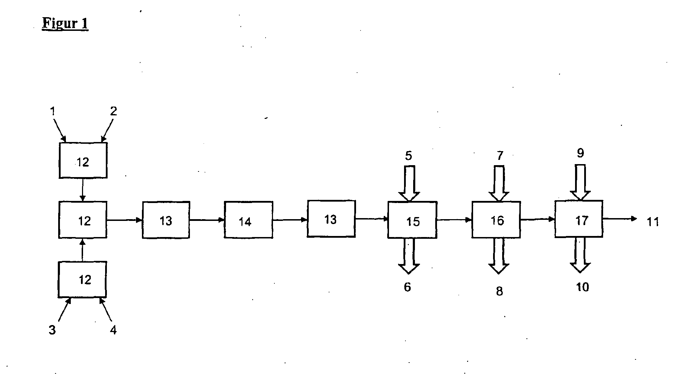

Die nachstehenden Ausführungsbeispiele wurden in einer Apparatur gemäß Figur 1 durchgeführt. Hierfür werden zunächst die Edukte (1) und (2) über einen Kaskadenmischer der Ehrfeld Mikrotechnik BTS GmbH (EMB), einem Split- und Recombinemischer mit 5 Kaskaden und einer maximalen Kanalweite von 500µm, als statischen Mikromischer (12) vermischt. Zu dieser Mischung wird Edukt (3) in einem Kaskadenmischer als weiteren Mikromischer (12) zugemischt. Gegebenenfalls wird zu (3) vor dieser Vermischung ein Phlegmatisierungsmittel (4) in einem Schlitzplattenmischer LH 25 der EMB mit lamellenartig angeordneten Kanälen mit einer Kanalweite von 25 bis 50 µm als statischen Mikromischer (12) zugemischt. Die so vorgemischten Edukte (1), (2), (3) und ggf. (4) durchlaufen dann ein Rohr-Temperiermodul der Ehrfeld Mikrotechnik BTS GmbH als Wärmetauscher (13), verbleiben anschließend in einer Kapillare als Verweilvolumen (14) und durchlaufen ggf. ein weiteres Rohr-Temperiermodul (13). Von dort durchlaufen sie die Extraktionsstufen (15), (16) und (17), wobei (15) und (16) aus einem Kaskadenmischer der Ehrfeld Mikrotechnik BTS GmbH, einem Settler (Abscheider) als Separator und ggf. einem Rohr-Temperiermodul der Ehrfeld Mikrotechnik BTS GmbH in unterschiedlicher Stufenzahl und die Trockenstufe (17) aus einem Kaskadenmischer der EMB und einem Settler (Abscheider) als Separator bestehen. Dabei wurden die folgenden Verbindungen/Temperaturen verwendet:

| **Nr.** | **Stoffstrom** | **Temperatur** |
|---|---|---|
| **1** | Base oder Säure | 0-25 °C |
| **2** | Hydroperoxid oder Wasserstoffperoxid | 10-25°C |
| **3** | Keton, Säurechlorid oder Chlorformiat | 10-25°C |
| **4** | Phlegmatisierungsmittel | 10-25°C |
| **5** | Wasser | 5-25°C |
| **6** | Mutterlauge der Reaktion | 0-25°C |
| **7** | Waschlösungen (bis zu 4 verschiedene) | 0-25°C |
| **8** | Abwasser aus Waschschritten (bis zu 4 Schritte) | 0-25°C |
| **9** | Getrocknete Luft | -15-0°C |
| **10** | Beladene Abluft | -10 - 25°C |
| **11** | Produkt | -10 - 25°C |
| **12** | Statische Mikromischer | |
| **13** | Wärmetauscher | |
| **14** | Verweilvolumen | |
| **15** | Extraktionsstufe aus Mischer, Separator und ggf. Wärmetauscher | |
| **16** | Extraktionsstufe aus Mischer, Separator und ggf. Wärmetauscher (bis zu 4 Stufen) | |
| **17** | Trocknungsstufe aus Mischer und Separator | |

### Beispiel 1: Herstellung von tert.- Butylperoxy-2-ethylhexanoat

Das tert.-Butylperoxy-2-ethylhexanoat wurde in einer Anlage, wie in Figur 1 dargestellt, mit einem Gesamtreaktionsvolumen von 75 ml hergestellt. Bei Temperaturen in der Reaktionszone von 40-55°C wurden nachstehende Versuchsparameter eingestellt:

| **Nr.** | **Stoffstrom** | **Temperatur** | **Massenstrom** |
|---|---|---|---|
| **1** | Kali- oder Natronlauge (10-50%) | 0-25°C | 4,2-21,1 g/min |
| **2** | tert.-Butylhydroperoxid (70-100 %) | 10-25°C | 3,0-4,5 g/min |
| **3** | 2-Ethylhexansäurechlorid | 10-25°C | 4,6 g/min |
| **7** | Natronlauge (10 %), Bisulfitlösung (10 %), Bicarbonatlösung (5 %) und Natriumacetatlösung (5 %) | 0-25°C | Je 8,0 g/min |
| **8** | Abwasser aus Waschschritten | 0-25°C | Je 8,0 g/min |
| **9** | Getrocknete Luft | -15-0°C | 6,0-8,0 g/min |
| **10** | Beladene Abluft | -10-25°C | 6,2-8,3 g/min |
| **11** | tert.-Butylperoxy-2-ethylhexanoat | -10 - 25°C | 6,1 g/min |

Das hergestellte tert.-Butylperoxy-2-ethylhexanoat konnte hierbei bei einer Massenausbeute größer als 90 % mit einer Produktqualität von oberhalb 99 % an Peroxidgehalt hergestellt werden.

### Beispiel 2: Herstellung von tert.- Butylperoxy-2-ethylhexanoat mittels Einsatz von Methylimidazol

Das tert.-Butylperoxy-2-ethylhexanoat unter Verwendung von 1-Methylimidazol wurde in einer Anlage, wie in Figur 1 dargestellt, mit einem Gesamtreaktionsvolumen von 25 ml hergestellt. Bei Temperaturen in der Reaktionszone von 25-35°C ohne Zugabe von Wasser vor der Reaktionszone wurden nachstehende Versuchsparameter eingestellt:

| **Nr.** | **Stoffstrom** | **Temperatur** | **Massenstrom** |
|---|---|---|---|
| **1** | 1-Methylimidazol | 0-25°C | 1,2-4,0 g/min |
| **2** | tert.-Butylhydroperoxid (70-100 %) | 10-25°C | 0,76-1,1 g/min |
| **3** | 2-Ethylhexansäurechlorid | 10-25°C | 1,54 g/min |
| **5** | Wasser | 5-25°C | 7,0-28,0 g/min |
| **6** | Mutterlauge der Reaktion | 0-25°C | 8,2-33,0 g/min |
| **7** | Natronlauge (10 %), Bisulfitlösung (10 %), Bicarbonat-lösung (5 %) und Natriumacetatlösung (5 %) | 0-25°C | Je 2,9 g/min |
| **8** | Abwasser aus Waschschritten | 0-25°C | Je 3,0 g/min |
| **9** | Getrocknete Luft | -15-0°C | 6,0-8,0 g/min |
| **10** | Beladene Abluft | -10-25°C | 6,2-8,3 g/min |
| **11** | tert.-Butylperoxy-2-ethylhexanoat | -10-25°C | 2,03 g/min |

Das hergestellte tert.-Butylperoxy-2-ethylhexanoat konnte hierbei bei einer Massenausbeute größer als 88 % mit einer Produktqualität von oberhalb 95 % an Peroxidgehalt hergestellt werden.

### Beispiel 3: Herstellung von Di-(2-ethylhexyl)-peroxydicarbonat

Das Di-(2-ethylhexyl)-peroxydicarbonat wurde in einer Anlage, wie in Figur 1 dargestellt, mit einem Gesamtreaktionsvolumen von 20 ml hergestellt. Bei Temperaturen in der Reaktionszone von 30-35°C wurden nachstehende Versuchsparameter eingestellt:

| **Nr.** | **Stoffstrom** | **Temperatur** | **Massenstrom** |
|---|---|---|---|
| **1** | Kali- oder Natronlauge (10-50 %) | 0-25°C | 0,84-2,1g/min |
| **2** | Wasserstoffperoxid (30-70 %) | 10-25°C | 0,2-0,6 g/min |
| **3** | 2-Ethylhexylchlorformiat | 10-25°C | 1,6 g/min |
| **4** | Isododekan | 10-25°C | 0,4-0,6 g/min |
| **5** | Wasser | | |
| **6** | Mutterlauge der Reaktion | 0-25°C | 1,26-2,9 g/mm |
| **7** | Bicarbonatlösung (5 %) | 0-25°C | 1,2 g/mn |
| **8** | Abwasser aus der Wäsche | 0-25°C | 1,3 g/min |
| **9** | Getrocknete Luft | -15-0°C | 4,1-5,5 g/min |
| **10** | Beladene Abluft | -10-25°C | 4,2-5,7 g/min |
| **11** | Di-(2-ethylhexyl)-peroxydicarbonat | -10-25°C | 1,38 g/min |

Das hergestellte Di-(2-ethylhexyl)-peroxydicarbonat konnte hierbei bei einer Massenausbeute größer als 92 % hergestellt werden. Das Produkt liegt als Abmischung in Isododekan vor mit einem Peroxidgehalt von 70-85 %.

### Beispiel 4: Herstellung von tert.-Butylperoxy-2-ethylhexylcarbonat

Das tert.-Butylperoxy-2-ethylhexylcarbonat wurde in einer Anlage, wie in Figur 1 dargestellt, mit einem Gesamtreaktionsvolumen von 25 ml hergestellt. Bei Temperaturen in der Reaktionszone von 55°C wurden nachstehende Versuchsparameter eingestellt:

| **Nr.** | **Stoffstrom** | **Temperatur** | **Massenstrom** |
|---|---|---|---|
| **1** | Kali- oder Natronlauge (10-50 %) | 0-25°C | 1,4-6,8 g/min |
| **2** | tert.-Butylhydroperoxid (70-100 %) | 10-25°C | 1,0-1,5 g/min |
| **3** | 2-Ethylhexylchlorformiat | 10-25°C | 1,7 g/min |
| **5** | Wasser | | |
| **6** | Mutterlauge der Reaktion | 0-25°C | 1,9-7,8 g/min |
| **7** | Natronlauge (10 %), Bisulfitlösung (10 %), Bicarbonat-lösung (5 %) und Natriumacetatlösung (5 %) | 0-25°C | Je 3,2 g/min |
| **8** | Abwasser aus 1. Wäsche | 0-25°C | Je 3,3 g/min |
| **9** | Getrocknete Luft | -15-0°C | 6,5-8,7 g/min |
| **10** | Beladene Abluft | -10-25°C | 6,6-8,8 g/min |
| **11** | tert.-Butylperoxy-2-ethylhexylcarbonat | -10-25°C | 2,17 g/min |

Das hergestellte tert.-Butylperoxy-2-ethylhexylcarbonat konnte hierbei bei einer Massenausbeute größer als 95 % mit einer Produktqualität von oberhalb 93 % an Peroxidgehalt hergestellt werden.

### Beispiel 5: Herstellung von 1,1-Di-(tert-butylperoxy)-3,3,5-trimethyl-cyclohexan

Das 1,1-Di-(tert.-butylperoxy)-3,3,5-trimethylcyclohexan wurde in einer Anlage, wie in Figur 1 dargestellt, mit einem Gesamtreaktionsvolumen von 45 ml hergestellt. Bei Temperaturen in der Reaktionszone von 12-18°C wurden nachstehende Versuchsparameter eingestellt:

| **Nr.** | **Stoffstrom** | **Temperatur** | **Massenstrom** |
|---|---|---|---|
| **1** | Schwefelsäure (50-98 %) | 0-20°C | 1,4-2,7 g/min |
| **2** | tert.-Butylhydroperoxid (70-100 %) | 10-20°C | 2,7-4,0 g/min |
| **3** | 3,3,5-Trimethylcyclohexanon | 10-20°C | 1,89 g/min |
| **5** | Mutterlauge der Reaktion | 0-25°C | 2,1-4,71 g/min |
| **6** - | Weichwasser, Natronlauge (10 %) | 0-20°C | 4,1 g/min und 3,9 g/min |
| **7** | Abwasser aus Waschschritten | 0-20°C | 4,2 g/min und 4,0 g/min |
| **8** | Getrocknete Luft | -15-0°C | 11,6-15,5 g/min |
| **9** | Beladene Abluft | -10-25°C | 11,9-16,0 g/min |
| **10** | 1,1-Di-(tert.-butylperoxy)-3,3,5-trimethylcyclohexan | -10-25°C | 3,88 g/min |

Das hergestellte 1,1-Di-(tert.-butylperoxy)-3,3,5-trimethylcyclohexan konnte hierbei bei einer Massenausbeute größer als 63 % mit einer Produktqualität von oberhalb 72 % an Peroxidgehalt hergestellt werden.

### Beispiel 6: Herstellung von Di-(3,5,5-trimethylhexanoyl)-peroxid

Das Di-(3,5,5-trimethylhexanoyl)-peroxid wurde in einer Anlage, wie in Figur 1 dargestellt, mit einem Gesamtreaktionsvolumen von 60 ml hergestellt. Bei Temperaturen in der Reaktionszone von 26-32°C wurden nachstehende Versuchsparameter eingestellt:

| **Nr.** | **Stoffstrom** | **Temperatur** | **Massenstrom** |
|---|---|---|---|
| **1** | Kali- oder Natronlauge (10-50 %) | 0-25 °C | 2,5-12,6 g/min |
| **2** | Wasserstoffperoxid (30-70 %) | 10-25°C | 0,6-1,6 g/min |
| **3** | 3,5,5-Trimethylhexanoylchlorid | 10-25°C | 2,7 g/min |
| **5** | Wasser | | |
| **6** | Mutterlauge der Reaktion | 0-25°C | 1,5-14,6 g/min |
| **7** | Natronlauge (10 %), Weichwasser | 0-25°C | 2,1 g/min und 1,5 g/min |
| **8** | Abwasser aus Waschschritten | 0-25°C | 2,3 g/min und 1,6 g/min |
| **9** | Getrocknete Luft | -15-0°C | 6,9-9,2 g/min |
| **10** | Beladene Abluft | -10-25°C | 7,0-9,4 g/min |
| **11** | Di-(3,5,5-trimethylhexanoyl)-peroxid | -10 25°C | 2,3 g/min |

Das hergestellte Di-(3,5,5-trimethylhexanoyl)-peroxid konnte hierbei bei einer Massenausbeute größer als 92 % mit einer Produktqualität von oberhalb 91 % an Peroxidgehalt hergestellt werden.

### Vergleich der Herstellung von Perestern in herkömmlichen Verfahren und mit Hilfe der Mikroreaktionstechnik

### Beispiel 7: Herstellung von tert.-Butylperpivalat

Tert.-Butylperpivalat wird im kontinuierlichen technischen Herstellungsprozess typischerweise in einer 3-stufigen Rührkesselkaskade mit einem Kesselvolumen von jeweils 350 Litern mit den nachfolgenden Parametern hergestellt:

| **Nr.** | **Stoffstrom** | **Temperatur** | **Massenstrom** |
|---|---|---|---|
| 1 | Kali- oder Natronlauge (45 %) | 20°C | 3,9 kg/min |
| 2 | tert.-Butylhydroperoxid (70 %) | 20°C | 3,5 kg/min |
| 3 | Pivalinsäurechlorid | 15°C | 2,7 kg/min |
| 4 | Isododekan | 20°C | 1,1 kg/min |
| 5 | Natronlauge (10 %), Bisulfitlösung (10 %), Bicarbonat-lösung (5 %) und Natriumacetatlösung (5 %) | 10°C | Je 5,8 kg/min |
| 6 | tert.-Butylperoxypivalat (75 %ig in Isododekan) | 8°C | 4,37 kg/min |

Die Ausbeute liegt bei dem herkömmlichen Prozess bei 84,0 % der Theorie.

Das tert.-Butylperpivalat wurde in einer mikroreaktionstechnischen Anlage, wie in Figur 1 dargestellt, mit einem Gesamtreaktionsvolumen von 60 ml hergestellt. Bei Temperaturen in der Reaktionszone von 14-21°C wurden nachstehende Versuchsparameter eingestellt:

| **Nr.** | **Stoffstrom** | **Temperatur** | **Massenstrom** |
|---|---|---|---|
| 1 | Kali- oder Natronlauge (10-50 %) 0 - | 20°C | 1,5 - 11,0 g/min |
| 2 | tert.-Butylhydroperoxid (70-.100 %) 10- | 20°C | 0,7 - 3,6 g/min |
| 3 | Pivalinsäurechlorid 0 - | 15°C | 2,7 g/min |
| 4 | Isododekan | 0-25°C | 0,6-1,1 g/min |
| 5 | Natronlauge (10 %), Bisulfitlösung (10 %), Bicarbonatlösung (5 %) und Natriumacetatlösung (5 %) | 10°C | Je 5,8 g/min |
| 6 | tert.-Butylperoxypivalat (75 %ig in Isododekan) | -10 - 20°C | 4,8 g/min |

Durch die Durchführung der Synthese in mikroreaktionstechnischen Anlagen konnte die Umsetzung bei der Reaktion deutlich verbessert werden. Das so hergestellte tert.-Butylperpivalat als 75 %ige Lösung in Isododekan konnte typischerweise mit einer Ausbeute von 92,9 % bezogen auf das eingesetzte Säurechlorid erhalten werden.

### Vergleich der Herstellung von Peroxydicarbonaten in herkömmlichen Verfahren und mit Hilfe der Mikroreaktionstechnik

### Beispiel 8: Herstellung von Isopropylperoxydicarbonat

Isopropylperoxydicarbonat wird im kontinuierlichen technischen Herstellungsprozess typischerweise in einer 2-stufigen Rührkesselkaskade mit einem Kesselvolumen von jeweils 80 Litern mit den nachfolgenden Parameter hergestellt:

| **Nr.** | **Stoffstrom** | **Temperatur** | **Massenstrom** |
|---|---|---|---|
| 1 | Natronlauge (32,3 %) | 10°C | 1,08 kg/min |
| 2 | Wasserstoffperoxid (20,5 %) | 10°C | 0,76 kg/min |
| 3 | Isopropylchlorformiat | 10°C | 1,02 kg/min |
| 4 | Diethylenglykol-bis-(allylcarbonat) | 5°C | 2,48 kg/min |
| 5 | Wäsche mit Wasser | 5°C | 1,36 kg/min |
| 6 | Isopropylperoxydicarbonat (20 %ig in Isododekan) | 5°C | 2,98 kg/min |

Die Ausbeute liegt bei dem herkömmlichen Prozess bei 94,0 % der Theorie. Die starke Hydrolyse des eingesetzten Isopropylchlorformiats gebietet es, den Wasseranteil im Reaktionsgemisch so gering wie möglich zu halten. Im herkömmlichen Reaktionsverfahren ist dies aus sicherheitstechnischen Gründen nicht realisierbar. Zudem enthält die Mischung von Isopropylperoxydicarbonat mit aushärtbaren optischen Monomeren, wie sie von den Herstellern optischer Kunststoffgläser verlangt wird, einen nicht tolerierbaren Gehalt an Chlorid von typischerweise 130-230 ppm.

Bei der Herstellung von Isopropylperoxydicarbonat in Mikroreaktionsanlagen, wie in Figur 1 dargestellt, mit einem Gesamtreaktionsvolumen von 95 ml erlaubt die sehr schnelle Durchmischung der Reaktionspartner sowie die effektive Abfuhr der Reaktionswärme eine Durchführung der Synthese bei 15°C mit erheblich weniger Wasser im Reaktionsgemisch. Es wurden die folgenden Versuchsparameter eingestellt:

| **Nr.** | **Stoffstrom** | **Temperatur** | **Massenstrom** |
|---|---|---|---|
| 1 | Natronlauge (45 %) | 10-25°C | 2,17 g/min |
| 2 | Wasserstoffperoxid (50 %) | 10-20°C | 0,85 g/min |
| 3 | Isopropylchlorformiat. | 10-20°C | 2,90 g/min |
| 4 | Diethylenglykol-bis-(allylcarbonat) | 5-15°C | 9,66 g/min |
| 5 | Wäsche mit Wasser | 5°C | 5,05 g/min |
| 6 | Isopropylperoxydicarbonat (20 %ig in Isododekan) | 5°C | 12,08 g/min |

Überraschenderweise wurde bei der Synthese in mikroreaktionstechnischen Anlagen ein IPP-Diethylenglykol-bis-(allylcarbonat)-Gemisch mit einer Peroxidausbeute über 99 % und einem Chloridgehalt von weniger als 10 ppm in einer 12 %igen Abmischung erhalten. Damit ist es gelungen, ein einfaches und sicheres Verfahren zur Herstellung von Isopropylperoxydicarbonat in Diethylenglykol-bis-(allylcarbonat) zu entwickeln, dass zudem den hohen Qualitätsansprüchen der Hersteller optischer Kunststofflinsen genügt.

### Vergleich der Herstellung von Ketonperoxiden in herkömmlichen Verfahren und mit Hilfe der Mikroreaktionstechnik

### Beispiel-9 : Herstellung- von -Methylisobutylketonperoxid

Methylisobutylketonperoxid wird im technischen Herstellungsprozess typischerweise in Batchansätzen von 600-900 kg Ansatzgröße bei 20-25°C und 2 bis 3 Stunden Reaktionszeit mit den nachfolgenden Parameter hergestellt:

| **Nr.** | **Stoffstrom** | **Temperatur** | **Masse** |
|---|---|---|---|
| 1 | Methylisobutylketon | 20°C | 160-290 kg |
| 2 | Wasserstoffperoxid (70 %) | 20°C | 270-490 kg |
| 3 | Schwefelsäure (78 %) | 20°C | 14-25 kg |
| 4 | Isododekan | 20°C | 80-120 kg |
| 5 | Methylisobutylketonperoxid (Isomerengemisch) | 20°C | 220-400 kg |

Das erhaltene Produkt weist bei dem herkömmlichen Prozess einen Aktivsauerstoff vom ca. 11,0 % auf.

Das Methylisobutylketonperoxid wurde in einer mikroreaktionstechnischen Anlage, wie in Figur 1 dargestellt, mit einem Gesamtreaktionsvolumen von 170 ml hergestellt. Bei Temperaturen in der Reaktionszone von 22-30°C wurden nachstehende Versuchsparameter eingestellt:

| **Nr.** | **Stoffstrom** | **Temperatur** | **Massenstrom** |
|---|---|---|---|
| 1 | Methylisobutylketon | 15-30°C | 4,8 g/min |
| 2 | Wasserstoffperoxid (70 %) | 20 - 25°C | 2,36 g/min |
| 3 | Schwefelsäure (96 %) | 10-25°C | 0,11 g/min |
| 4 | Isododekan . | 5 - 30°C | 2,2 g/min |
| 5 | Methylisobutylketonperoxid (Isomerengemisch) | 20-25°C | 11,22 g/min |

Man erhält ein Produkt mit ca. 11,6 % Aktivsauerstoff. Durch die Durchführung der Synthese in mikroreaktionstechnischen Anlagen konnte die Umsetzung der Reaktanden zum Ketonperoxid bei 25°C und 5,8 Minuten Verweilzeit um 47 % gesteigert werden, obwohl die eingesetzte Menge an Wasserstoffperoxid im Verhältnis zum Keton um 50 % gegenüber dem herkömmlichen Verfahren reduziert worden ist.

### Vergleich der Herstellung von tert.-Butylperoxycarbonaten in herkömmlichen Verfahren und mit Hilfe der Mikroreaktionstechnik

### Beispiel 10: tert.-Butylperoxyisopropylcarbonat

Tert.-Butylperoxyisopropylcarbonat wird im kontinuierlichen technischen Herstellungsprozess typischerweise in einer 3-stufigen Rührkesselkaskade mit einem Volumen von jeweils 350 Litern mit den nachfolgenden Parametern hergestellt:

| **Nr.** | **Stoffstrom** | **Temperatur** | **Massenstrom** |
|---|---|---|---|
| 1 | Kalilauge (45%) | 15°C | 3,3 kg/min |
| 2 | tert.-Butylhydroperoxid (70 %) | 15°C | 3,3 kg/min |
| 3 | Isopropylchlorformiat | 10°C | 2,3 kg/min |
| 4 | Isododekan | 15°C | 0,7 kg/min |
| 5 | Weichwasser | 15°C | 4,0 kg/min |
| 6 | Natronlauge (10 %), Bisulfitlösung (10 %), Bicarbonat-lösung (5 %) und Natriumacetatlösung (5 %) | 10°C | Je 6,1 kg/min |
| 7 | tert.-Butylperoxyisopropylcarbonat (75 %ig in Isododekan) | 10°C | 3,71 kg/min |

Die Ausbeute liegt bei dem herkömmlichen Prozess bei 84,0 % der Theorie.

Das tert.-Butylperoxyisopropylcarbonat wurde in einer mikroreaktionstechnischen Anlage, wie in **Figur 1** dargestellt, mit einem Gesamtreaktionsvolumen von 175 ml hergestellt. Bei Temperaturen in der Reaktionszone von 19-31°C wurden nachstehende Versuchsparameter eingestellt:

| **Nr.** | **Stoffstrom** | **Temperatur** | **Massenstrom** |
|---|---|---|---|
| 1 | Kalilauge (45 %) | 15-25°C | 4,11 g/min |
| 2 | tert.-Butylhydroperoxid (70 %) | 15 - 25°C | 4,12 g/min |
| 3 | Isopropylchlorformiat | 10-25°C | 3,68 g/min |
| 4 | Isododekan | 15-25°C | 0,70 g/min |
| 5 | Weichwasser | 15-25°C | 2,00 g/min |
| 6 | Natronlauge (10 %), Bisulfitlösung (10 %), Bicarbonat-lösung (5 %) und Natriumacetatlösung (5 %) | 10 - 20°C | Je 7,3 g/min |
| 7 | tert.-Butylperoxyisopropylcarbonat (86 %ig in Isododekan) | 10 - 20°C | 6,09 g/min |

Der eingesetzte Überschuss an Hydroperoxid konnte bei diesem Verfahren gegenüber der herkömmlichen Methode um 28 % reduziert werden. Durch die Reduzierung des Wasseranteils im Reaktionsgemisch und die damit verbundene geringere Hydrolyse des eingesetzten Isopropylchlorformiats konnte bei der Herstellung in mikroreaktionstechnischen Anlagen ein tert.-Butylperoxy-isopropylcarbonat mit 99 % Peroxidausbeute erhalten werden.

## Patentansprüche

1. Verfahren zur Herstellung von organischen Peroxiden unter Einsatz von Wasserstoffperoxid oder Hydroperoxid, mindestens einer Base oder Säure und mindestens eines Ketons, Alkohols, Säurechlorids/-anhydrids und/oder Chlorformiat, **dadurch gekennzeichnet, dass** dieses in mindestens einem statischen Mikromischer durchgeführt wird.

2. Verfahren nach Anspruch 1, **dadurch gekennzeichnet, dass** als statische Mischer Multilaminationsmischer, Split und Recombinemischer, Caterpillarmischer oder auch Mischer mit einer Querschnittsverengung eingesetzt werden.

3. Verfahren nach einem der Ansprüche 1 oder 2, **dadurch gekennzeichnet, dass** der statische Mikromischer kontinuierlich durchströmt wird, die Reaktionsmischung mittels Wärmetauscher auf die geeignete Temperatur gebracht wird und anschließend gegebenenfalls die Reaktionsmischung in ein temperierbares Verweilvolumen eingespeist wird, dort eine durch das Volumen der Verweilstruktur und die Fließgeschwindigkeit der Reaktionsmischung vorbestimmte Zeit verbleibt, wobei in diesem Verweilvolumen die Ausgangsstoffe, die gegebenenfalls als nicht mischbare Phasen vorliegen, ständig miteinander intensiv vermischt werden.

4. Verfahren nach Anspruch 3, **dadurch gekennzeichnet, dass** das Verweilvolumen in Form eines statischen Mischers vorliegt.

5. Verfahren nach Anspruch 3 oder 4, **dadurch gekennzeichnet, dass** die Durchmischung der Ausgangsverbindungen im Verweilvolumen durch direkte oder indirekte Einwirkung von hochfrequenten Schwingungen bewirkt wird.

6. Verfahren nach einem oder mehreren der Ansprüche 1 bis 5, **dadurch gekennzeichnet, dass** ein Mikromischer mit Kanalweiten von kleiner als 200 µm eingesetzt wird.

7. Verfahren nach einem oder mehreren der Ansprüchen 1 bis 6, **dadurch gekennzeichnet, dass** es sich bei dem eingesetzten Wärmetauscher um einen mikrostrukturierten Wärmetauscher handelt.

8. Verfahren nach einem oder mehreren der Ansprüche 1 bis 7, **dadurch gekennzeichnet, dass** es sich bei der eingesetzten Verweilvolumen um eine Kapillare handelt.

9. Verfahren nach einem oder mehreren der Ansprüche 1 bis 8, **dadurch gekennzeichnet, dass** bei dem eingesetzten Verweilvolumen die Reaktionsmischung im Kreislauf umgepumpt wird, wobei gegegebenfalls ein statischer Mikromischer in den Kreislauf eingefügt ist.

10. Verfahren nach den vorgenannten Ansprüchen 1 bis 9, **dadurch gekennzeichnet, dass** der Temperaturverlauf im strömenden Reaktionsgemisch entlang der Strömungsrichtung durch eine Abfolge von Wärmetauschern und Verweilerstrecken einstellbar ist.

## Claims

1. Process for preparing organic peroxides using hydrogen peroxide or hydroperoxide, at least one base or acid and at least one ketone, alcohol, acid chloride/anhydride and/or chloroformate, **characterized in that** it is performed in at least one static micromixer.

2. Process according to Claim 1, **characterized in that** the static mixers used are multilamination mixers, split and recombine mixers, caterpillar mixers or else mixers with a cross-sectional constriction.

3. Process according to either of Claims 1 and 2, **characterized in that** the static micromixer is flowed through continuously, the reaction mixture is brought to the suitable temperature by means of heat exchangers and then the reaction mixture is optionally fed into a heatable delay volume, remains there for a time determined by the volume of the delay structure and the flow rate of the reaction mixture, and the starting materials which may be present as immiscible phases are constantly mixed intensively with one another within this delay volume.

4. Process according to Claim 3, **characterized in that** the delay volume is in the form of a static mixer.

5. Process according to Claim 3 or 4, **characterized in that** the mixing of the starting compounds in the delay volume is brought about by direct or indirect action of highfrequency vibrations.

6. Process according to one or more of Claims 1 to 5, **characterized in that** a micromixer with channel widths of less than 200 µm is used.

7. Process according to one or more of Claims 1 to 6, **characterized in that** the heat exchanger used is a microstructured heat exchanger.

8. Process according to one or more of Claims 1 to 7, **characterized in that** the delay volume used is a capillary.

9. Process according to one or more of Claims 1 to 8, **characterized in that** the reaction mixture is pumped in circulation in the delay volume used, and a static micromixer is optionally included in the circuit.

10. Process according to the above Claims 1 to 9, **characterised in that** the temperature profile in the flowing reaction mixture along the flow direction is adjustable by virtue of a sequence of heat exchangers and delay zones.

## Revendications

1. procédé de fabrication de peroxydes organiques utilisant du peroxyde d'hydrogène ou de l'hydroperoxyde, au moins une base ou un acide et au moins une cétone, un alcool, un chlorure/anhydride d'acide et/ou un chloroformiate, **caractérisé en ce que** celui-ci est réalisé dans au moins un micromélangeur statique.

2. Procédé selon la revendication 1, **caractérisé en ce que** des mélangeurs à multilaminage, des mélangeurs à partage et recombinaison, des mélangeurs à chenilles ou également des mélangeurs à rétrécissement de section sont utilisés en tant que mélangeur statique.

3. Procédé selon l'une quelconque des revendications 1 à 2, **caractérisé en ce que** le micromélangeur statique est traversé en continu, le mélange réactionnel est porté à la température appropriée par un échangeur de chaleur, puis le mélange réactionnel est éventuellement introduit dans un volume de séjour conditionnable, y reste pendant une durée prédéterminée par le volume de la structure de séjour et la vitesse d'écoulement du mélange réactionnel, les matières premières, qui se présentent éventuellement sous la forme de phases non mélangeables, étant mélangées intensivement les unes avec les autres en continu dans ce volume de séjour.

4. Procédé selon la revendication 3, **caractérisé en ce que** le volume de séjour se présente sous la forme d'un mélangeur statique.

5. Procédé selon la revendication 3 ou 4, **caractérisé en ce que** le mélange des composés de départ dans le volume de séjour est provoqué par l'action directe ou indirecte d'oscillations de haute fréquence.

6. Procédé selon une ou plusieurs des revendications 1 à 5, **caractérisé en ce qu'**un micromélangeur ayant des largeurs de canaux inférieures à 200 µm est utilisé.

7. Procédé selon une ou plusieurs des revendications 1 à 6, **caractérisé en ce que** l'échangeur de chaleur utilisé est un échangeur de chaleur microstructuré.

8. Procédé selon une ou plusieurs des revendications 1 à 7, **caractérisé en ce que** le volume de séjour utilisé est un capillaire.

9. Procédé selon une ou plusieurs des revendications 1 à 8, **caractérisé en ce que** le mélange réactionnel est pompé en circuit dans le volume de séjour utilisé, un micromélangeur statique étant éventuellement inséré dans le circuit.

10. Procédé selon les revendications 1 à 9 précédentes, **caractérisé en ce que** l'évolution de température dans le mélange réactionnel en écoulement le long de la direction d'écoulement est ajustable par une succession d'échangeurs de chaleur et de sections de séjour.
